# EUROPEAN PATENT APPLICATION

(11) **EP 0 919 259 A1**
(43) Date of publication of application: **02.06.1999**
(21) Application number: 98307463.4
(22) Date of filing: 15.09.1998
(51) Int. Cl.: A63B 24/00

(54) **System for controlling and coordinating exercise equipment**

(30) Priority: 25.11.1997 GB 9724924
(71) Applicant: Cybersport Limited, Kingston-upon-Thames, Surrey KT2 6HE (GB)
(72) Inventor: Spalding, Andrew, Crouch Emd, London N8 8EG (GB); Wales, Frank, Chiswick, London W4 4PH (GB)
(74) Representative: Bucks, Teresa Anne

(57) **Abstract**

The invention relates to a system for co-ordinating and controlling exercise equipment across telecommunications networks and apparatus for controlling a mechanical exercise device to operate in a predetermined exercise procedure with other similar such devices. The exercise devices each include sensors to provide signals representing selected performance parameters of the device and transducers responsive to control signals for setting selected operating parameters of the device. The apparatus comprise a local processor for connection to said exercise device to receive said sensor signals and to generate and send said control signals. The local processor has a network interface enabling the local processor to communicate over a network with a remote central processor, whereby the central processor can communicate with a plurality of said control apparatuses to co-ordinate the control of respective said exercise devices to operate in the exercise procedure.

## Description

The invention relates to a system for co-ordinating and controlling exercise equipment across telecommunications networks.

In an effort to remain fit and healthy many people endeavour to take frequent exercise, some belong to health and fitness clubs where they can enjoy exercise in the company of other like-minded people and they can train and indeed race together which helps to keep them motivated. Unfortunately in this day and age many people are too busy to take time out to visit such clubs and others find the membership fees too costly. These people therefore rely on the use of aerobic home exercise equipment such as rowing machines, exercise bikes and treadmills. However, it is acknowledged that exercise at home alone can be a very boring and solitary activity. With the lack of feedback, no interaction and no fun, good intention to carry out regular exercise frequently falls by the wayside in as short a time as three weeks. Some people endeavour to avoid this problem by buying exercise videos to give the illusion that they are exercising with other people or by hiring personal trainers to provide interaction and feedback.

The makers of home exercise equipment have also tried to address this problem by providing equipment which utilises digital graphics, interactive software and display to engage the user's attention together with exercise and steering mechanisms to stretch the user physically. One example of such interactive exercise apparatus is described in International Patent Specification WO-A-94/17860. This describes an exercise cycling apparatus controlled by a computer to run various interactive programmes to provide a variety of environments, e.g. roads, terrain and obstacles for the user, underwater adventure, pedal-powered flight simulators and space travel. The cycling apparatus provides the computer with information such as user weight, pedal speed, steering or tilt information which is used to update the user's position and direction within the computer simulated environment. The computer equally controls the apparatus, such as pedal resistance, to simulate certain elements, e.g. hills, gear changes, road surfaces etc.

It is suggested in the specification that a number of computers from several different pieces of exercise apparatus can be interconnected via a network interface module so that multiple users can exercise in the same simulated environment to interact as team mates or competitors. However, the networking can only be between apparatus which allows a user to indicate a desired direction of motion, i.e. by steering and which already includes a computer or processor to allow interconnection and communication with other such exercise machines. Thus, this prior art system has limited application.

It is an object of the present invention to provide a system for co-ordinating and controlling any type of exercise equipment across telecommunications or other networks so that owners of exercise equipment including rowing machines, exercise bikes, treadmills, stair apparatus, water rowers and so on can train and race with each other from remote locations in real-time over a telecommunications network.

The invention therefore provides apparatus for controlling a mechanical exercise device to operate in a predetermined exercise procedure with other similar such devices, the exercise devices each including sensors to provide signals representing selected performance parameters of the device and transducers responsive to control signals for setting selected operating parameters of the device, the apparatus comprising a local processor for connection to said exercise device to receive said sensor signals and to generate and send said control signals,
said local processor having a network interface enabling the local processor to communicate over a network with a remote central processor, whereby the central processor can communicate with a plurality of said control apparatuses to co-ordinate the control of respective said exercise devices to operate in the exercise procedure,
the local processor being arranged to communicate with the remote central processor first tier data comprising at least data sent to the central processor identifying the local processor, data sent to the local processor defining the essential parameters of the exercise procedure, data sent to the central processor defining final result parameters of the exercise procedure performed on the respective exercise device, and data sent to the local processor defining final result parameters of the exercise procedure performed on the other exercise devices,
the local processor being arranged also to communicate with the remote central processor second tier data comprising at least data sent to the central processor during the course of the exercise procedure representing intermediate parameters of the exercise procedure performed on the respective exercise device, and data sent to the local processor during the course of the exercise procedure representing said intermediate parameters of the exercise procedure performed on the other exercise devices,
wherein the local processor is arranged to communicate said first tier data using a first protocol employing sequenced and acknowledged data packets and is arranged to communicate said second tier data using a second protocol employing unacknowledged data packets.

The invention further provides an exercise system comprising a plurality of mechanical exercise devices controlled to operate in a predetermined exercise procedure,
each exercise device including sensors to provide signals representing selected performance parameters of the device and transducers responsive to control signals for setting selected operating parameters of the device,
a local processor connected to each exercise device to receive said sensor signals and to generate and send said control signals,
said local processors each having a network interface communicating over a network with a remote central processor, whereby the central processor communicates with the plurality of local processors to coordinate the control of the respective exercise devices to operate in the exercise procedure,
the local processors being arranged to communicate with the remote central processor first tier data comprising at least data sent to the central processor identifying the local processor, data sent to the local processors defining the essential parameters of the exercise procedure, data sent to the central processor defining final result parameters of the exercise procedure performed on the respective exercise devices, and data sent to the local processors defining final result parameters of the exercise procedure performed on the plurality of exercise devices,
the local processors being arranged also to communicate with the remote central processor second tier data comprising at least data sent to the central processor during the course of the exercise procedure representing intermediate parameters of the exercise procedure performed on the plurality of exercise devices, and data sent to the local processors during the course of the exercise procedure representing said intermediate parameters of the exercise procedure performed on the plurality of exercise devices,
the local processors being arranged to communicate said first tier data using a first protocol employing sequenced and acknowledged data packet and further being arranged to communicate said second tier data using a second protocol employing unacknowledged data packets.

The invention also provides a method of controlling a plurality of mechanical exercise devices to operate in a predetermined exercise procedure, the exercise devices each including sensors to provide signals representing selected performance parameters of the device and transducers responsive to control signals for setting selected operating parameters of the device, the exercise devices each having a local processor connected to said exercise device to receive said sensor signals and to generate the sensor control signal, comprising the steps of
providing a means of communication between the local processor over a network with a remote central processor, whereby the central processor communicates with the plurality of local processors to coordinate the control of the plurality of exercise devices to operate in the exercise procedure,
the local processors communicating with the remote central processor first tier data comprising at least data sent to the central processor identifying the local processor, data sent to the local processor defining the essential parameters of the said exercise procedure, data sent to the central processor defining final result parameters of the exercise procedure performed on the respective exercise devices, and data sent to the local processor defining final result parameters of the exercise procedure performed on the other exercise devices,
the local processor communicating with the remote central processor second tier data comprising at least data sent to the central processor during the course of the exercise procedure representing intermediate parameters of the exercise procedure performed on the respective exercise devices, and data sent to the local processor during the course of the exercise procedure representing said intermediate parameters of the exercise procedure performed on the other exercise devices,
wherein the local processor is arranged to communicate said first tier data using a first protocol employing sequenced and acknowledged data packets and is arranged to communicate said second tier data user using a second protocol employing unacknowledged data packets.

A preferred embodiment of the invention will now be described, by way of example only, with reference to the accompanying drawings in which:-
Fig. 1 is a block diagram showing an overview of the communication between the apparatus of the present invention and the external components required for operating a number of mechanical exercise devices in a predetermined exercise procedure;
Fig. 2 is a flow chart illustrating the interaction between the apparatus of the present invention and the server computer.

The invention comprises apparatus for controlling a mechanical exercise device to operate in a predetermined exercise procedure with other such devices. This effectively provides an online health and fitness "club" for people with independent exercise equipment, such as rowing machines, water rowers, exercise bikes, treadmills and stair machines. It facilitates interactive training or racing, so that people (hereinafter the user or users) in remote locations, such as at home or in different clubs, can train or race with others to provide personal motivation and coaching.

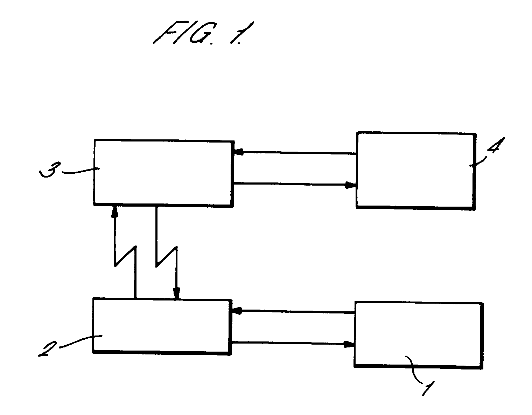

Referring to Fig. 1 the sports or exercise equipment 1 must be a mechanical device on which the user can perform exercise. The device can be for rowing, cycling, climbing, skiing, walking or running. The equipment 1 must incorporate sensors which enable signals (sensor signals) representing performance data to be sent via an appropriate connection, such as an RS232 cable, to local processing means provided by the user's computer 2. The equipment 1 should also have transducers responsive to control signals passed form the user's computer 2 for setting selected operating parameters of the equipment 1, such as the dynamic loading. The user's computer 2 is located at the site of the exercise equipment 1, and is preferably an IBM compatible PC which runs the user's software. The user's computer 2 has a network interface which enables it to be linked to a remote central processor, the server 3, via a network, which can be accessed by a number of different user computers 2. The network can be the internet, a local area network (LAN) or a wide area network (WAN). The user requires software which enables the user's computer to communicate with the internet or other type of network.

The server 3 is programmed to perform a number of functions including the handling of communications with individual users, providing data on requests by user's computer 2, managing the registration of users for training events, controlling the scheduling of training event activities and other housekeeping activities that tie the user's activities into the user's records in a central database 4.

The server software includes a training event management process that is the first point of contact for all users using the system which interrogates the database 4 for information on users, schedules and so forth; it creates referee processes as appropriate to oversee a particular training event; it calculates the results of a training event (based on summary information presented by the user's computer 2) and updates the database 4 with the results of training activity.

The user's computer 2 performs a number of functions such as collecting data from the sports equipment 1; registering for a training event; modifying training event data with a user's handicap based on previous performance; sending training event data to the server 3; receiving training event data from other competitors via the server 3; interpolating missing training event data; monitoring the user's heart rate; comparing user's performance with previous training events and providing appropriate coaching messages; sending information to sports equipment 1 based on the position of the user on the training course and the location of other competitors to affect the dynamic loading of the exercise equipment e.g. to give the effect of slipstreaming or uphill work in the case of cycling; logging training event data; sending a summary of the training event to the server 4; and re-planning training events, allowing the user to train again without being connected to the server 4.

The user's computer 2 is programmed to provide a graphical user interface i.e. with software that handles the interaction with the user's computer 2, a communication module to talk to the remote server 3, and a hardware interface which enables the user's computer 2 to be attached to whatever sports equipment 1 the user has.

The user's computer 2 is therefore required to exchange a number of different types of information with theserver 3 and the software with which the user's computer 2 is programmed uses two different methods of transmitting data according to the nature of the data.

In a preferred embodiment the transmission methods are defined by two known communication protocols, the first being the Transmission Control Protocol (TCP)and the second being the User Datagram Protocol (UDP)of which special versions exist for use in software communicating via the internet.

TCP provides the ability to transmit sequenced and acknowledged packets of information and in effect establishes a virtual circuit between the server 3 and the user's computer 2. TCP is therefore used to provide a robust, serialised channel for exchanging key information, hereinafter referred to as first tier data, which ensures the successful handling of the race, such as logging in at the start of a training event, details of the course or race parameters or the results of a training event at the end.

UDP provides the ability to transmit rapid unacknowledged packets of information which are not sequenced. UDP is fast because the transport software managing the transmission is "very light". Messages are not queued or retried so there is no guarantee of delivery. The nature of this protocol is such that the information quickly decays in value, but it is more important that the general flow of information continues in as timely fashion as possible, albeit with the occasional glich, then the information is all received eventually. The UDP messages are given a time sequence number and any data received out of sequence is discarded. Thus UDP is used to exchange short lived information which should be delivered as soon as possible or not at all, such as countdowns and interval-by-interval race or training information during an event. The rapidity of the flow of information is such that the messages which are sent but not received are so quickly superseded that it does not matter. The vital information is sent via TCP as it is guaranteed to get there.

The following chart lists the protocol channel used for the transmission of certain types of information:-

| DESCRIPTION OF INFORMATION | PROTOCOL |
|---|---|
| Messages sent by the server to the users during the pre-race period to count down to the start of the race | UDP |
| Data about what the user did during the last interval | UDP |
| The User's summary of how it performed in the race | TCP |
| Data sent in response to a user's request for a particular piece of data from the server | TCP |
| User's messages requesting particular data from the server | TCP |
| User announcement that it is dropping out of the race | TCP & UDP |
| Server request that the message be displayed by the user | TCP |
| Cancellation of race announcement by the server | TCP |
| Information on the specific race (participant list, duration, etc.) | TCP |
| End of race announcement by the server | TCP & UDP |
| Registration acceptance from the server | TCP |
| Data broadcast about what the server received from users during the last interval | UDP |
| Registration rejection by the server | TCP |
| Registration request from the user | TCP |
| Information on the race course | TCP |
| The definitive results of a race | TCP |
| Start of race announcement by the server | TCP & UDP |
| The Server's summary of the whole race | TCP |
| Responses to a Status Request message | TCP |
| Requests for the status of the recipient | TCP |
| User requests that a message be sent to a list of other users | TCP |

The user's computer 2 and server 3 have the ability to allow either end to fill in gaps in the information sent on the UDP channel. The results of an event are not compromised, since the user's computer will upload summaries of how the user performed at the end of the event on the TCP channel and that information will be used by the server 3 to arrive at the definitive results of the race or training event.

The expected data transfer rate is preferably one UDP packet each way per second, with the user to server packet containing about ten to fifteen bytes of data and the server to user packet containing fifty to a hundred bytes. The principle loading at the server end is in the timely delivery of the burst of UDP packets sent out at the end of each racing interval, with, say, one hundred bytes per active user being sent out to the network.

However, the TCP messages are not sent at regular intervals. For example, in setting up a race there will be 2-3 messages; during the race there will be a message as each checkpoint is passed, say every 1-2 minutes.

The TCP packet sizes are generally about ten to one thousand bytes between the client and server and vice versa.

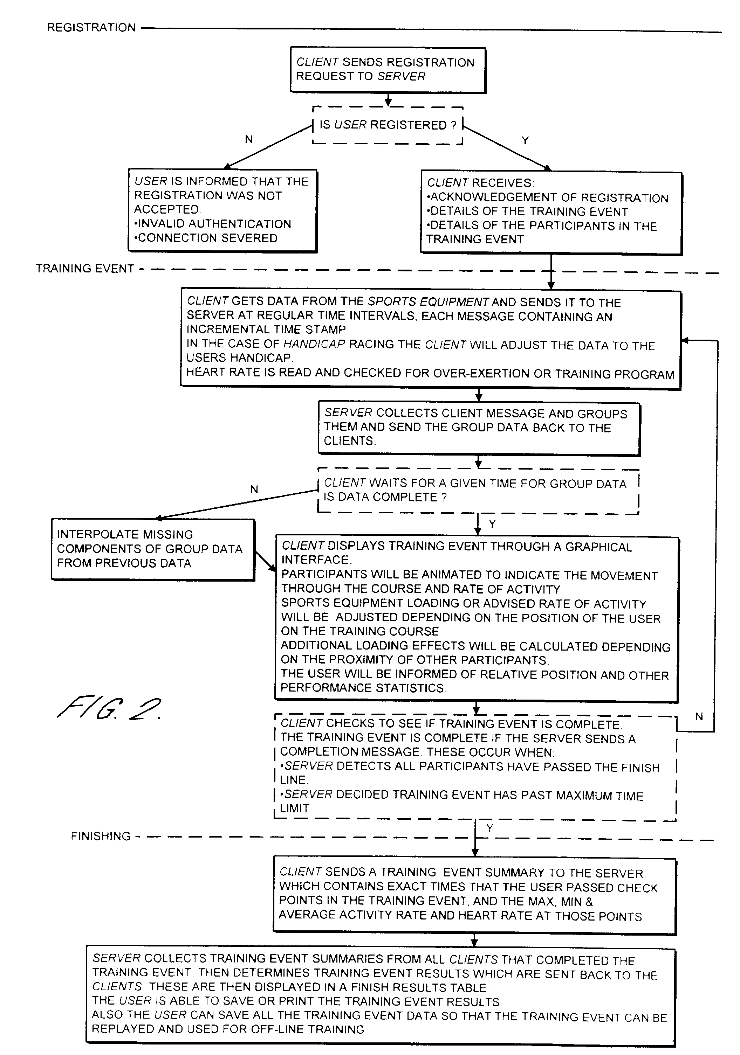

The system operation will now be described with reference to the flow chart of Fig. 2.

### Online Racing

In order to compete in an online race, the user must book a place in a scheduled race in advance. To register for a race, the user's computer 2 opens a network connection for communicating with the server 3 and sends a registration request to the server 3 and awaits a response. If the user is not booked into any upcoming races, the user's computer 2 will receive a rejection message from the server 3, in which case the user will not be taking part in the race, and the user's computer 2 will terminate the network connection with the server 3. If the user is accepted for a race, the server 3 will then send information to the user's computer 2 regarding the next race for which the user is booked. The user's computer 2 will wait for the server 3 to send the race profile, which specifies the distance of the race, the number of stages and checkpoint distances. Next, the user's computer 2 waits for the server 3 to send race information, which gives details on the other competitors in the race. Finally, to complete the registration phase, the user's computer 2 awaits the start of the race. During this time, the server 3 sends a countdown to each of the users involved in the current race.

At the end of the countdown, the race starts. During the race, the user's computers 2 acquire data from the exercise equipment 1 e.g. distance travelled, activity rate and heart rate, and sends it to the server 3 along with the time interval. The protocol used for this information flow is the UDP. The server 3 updates the positions of each of the competitors, and sends the race information to each of the user's computers 2 also by UDP. The user's computer 2 acts on this information by writing the details in the race summary, and updating the racer positions and user statistics from the screen display.

During the race, the user's computer 2 displays various information in a comments window, such as current position in the race, warning messages for the user's heart rate exceeding predefined limits, checkpoints reached, and comparisons against personal best time.

The race ends for the user when his (or her) racer crosses the finish line. While the other racers are finishing (if the user is not last), the user is given the option to type in comments about his race performance and the user's computer 2 then uploads its race summary and user comments to the server 3 and waits to receive the final results and overall race summary from the server 3 all via TCP. When the final race results have been received, the user's computer 2 displays these results, and the user is given the option to save the overall race summary (for the purposes of off-line training). A reference to the race summary will be stored as a means to re-run or view the last race. If the user bettered his personal best race time, the race summary will also be referred to as a personal best race.

At any point in the race, the user can elect to leave the race. The user's computer 2 sends a message via TCP to the server 3, and the user will be given the option to either spectate or exit so that no further information is received from the server 3 pertaining to that race. Spectating allows the user to carry on viewing the race without actually taking part. The race can also be cancelled by the server 3 at any time, in which case a race abandoned message will be sent to each user's computer 2 via TCP and UDP.

In the event of the network connection being lost, the server 3 will estimate the data which should have come from the user. The estimate will be an average based on previous values received from the user's computer 2. The server 3 then sends this data to each of the other users as normal. Similarly, if the user's computer 2 does not receive any data from the server 3 within an expected time period, the user's computer will update the positions of the other racers based on their average values, while attempting to restore the network connection with the server 3.

Transmitted race data is held in a log, which can be used for race replay and off-line racing.

### Off-line Racing

This is similar to online racing, except that no connection is made to the server 3 during racing. The user is able to compete in a previously saved race, and is then given the option to upload the race summary of the server 3 at the end of the race. The server maintains a league table of off-line racers, which is updated weekly.

Once the user has selected the required race, there will be a short countdown (to allow the user to get in position on the exercise equipment) and the race begins. At any point in the race, the user can elect to leave the race and return control to the main menu. During the race, the user's computer 2 must acquire data from the exercise equipment 1 (distance travelled, speed) and read data from the race summary in order to update the users statistics and positions of each of the competitors on the screen display.

The race ends for the user when his racer crosses the finish line. The user's computer 2 then gives the user the option to type in comments about their race performance and to upload their race summary and comments to the server 3. The off-line training session is now over.

### Race Replay

This allows the user to view a previously saved race without competing. Once the user has selected the required race, the race begins in reply mode. At any point during the race replay, the user can elect to quit. During the replay, the user's computer 2 must read data from the race summary and update the users statistics and positions of each of the competitors on the screen display. Replay mode ends when the last racer crosses the finish line.

### Save Race

The user can save the details of the entire race, i.e. the overall race summary for the purposes of off-line training and race replays by writing the overall race summary to disk.

### Save and Print Race Summary

The user can save the summary of the race, e.g. minimum, maximum and average rate, speed, heart rate and position etc. at each checkpoint by writing it to disk and this can then be printed together with user comment.

## Claims

1. Apparatus for controlling a mechanical exercise device (1) to operate in a predetermined exercise procedure with other similar such devices, the exercise devices each including sensors to provide signals representing selected performance parameters of the device and transducers responsive to control signals for setting selected operating parameters of the device, the apparatus comprising a local processor (2) for connection to said exercise device to receive said sensor signals and to generate and send said control signals,
said local processor having a network interface enabling the local processor to communicate over a network with a remote central processor (3), whereby the central processor can communicate with a plurality of said control apparatuses to co-ordinate the control of respective said exercise devices to operate in the exercise procedure,
characterised in that the local processor as arranged to communicate with the remote central processor first tier data comprising at least data sent to the central processor identifying the local processor, data sent to the local processor defining the essential parameters of the exercise procedure, data sent to the central processor defining final result parameters of the exercise procedure performed on the respective exercise device, and data sent to the local processor defining final result parameters of the exercise procedure performed on the other exercise devices,
the local processor being arranged also to communicate with the remote central processor second tier data comprising at least data sent to the central processor during the course of the exercise procedure representing intermediate parameters of the exercise procedure performed on the respective exercise device, and data sent to the local processor during the course of the exercise procedure representing said intermediate parameters of the exercise procedure performed on the other exercise devices,
wherein the local processor is arranged to communicate said first tier data using a first protocol employing sequenced and acknowledged data packets and is arranged to communicate said second tier data using a second protocol employing unacknowledged data packets.

2. Apparatus as claimed in claim 1 in which components of the first and second tier data are communicated using both protocols.

3. Apparatus as claimed in claim 1 or claim 2 in which the network is a local area network, a wide area network or the internet.

4. Apparatus as claimed in any one of the preceding claims in which the first protocol is a transmission control protocol and the second protocol is a user datagram protocol.

5. Apparatus as claimed in claim 1 further comprising a database (4) with which the central processor (3) is arranged to communicate to store or retrieve information relating, for example, to users of said exercise devices, paramaters relating to said exercise procedure and historical data relating to previous exercise procedures.

6. An exercise system comprising a plurality of mechanical exercise devices (1) controlled to operate in a predetermined exercise procedure,
each exercise device including sensors to provide signals representing selected performance parameters of the device and transducers responsive to control signals for setting selected operating parameters of the device,
a local processor (2) connected to each exercise device to receive said sensor signals and to generate and send said control signals,
said local processors each having a network interface communicating over a network with a remote central processor (3), whereby the central processor communicates with the plurality of local processors to coordinate the control of the respective exercise devices to operate in the exercise procedure,
characterised in that the local processors are arranged to communicate with the remote central processor first tier data comprising at least data sent to the central processor identifying the local processor, data sent to the local processors defining the essential parameters of the exercise procedure, data sent to the central processor defining final result parameters of the exercise procedure performed on the respective exercise devices, and data sent to the local processors defining final result parameters of the exercise procedure performed on the plurality of exercise devices,
the local processors being arranged also to communicate with the remote central processor second tier data comprising at least data sent to the central processor during the course of the exercise procedure representing intermediate parameters of the exercise procedure performed on the plurality of exercise devices, and data sent to the local processors during the course of the exercise procedure representing said intermediate parameters of the exercise procedure performed on the plurality of exercise devices,
the local processors being arranged to communicate said first tier data using a first protocol employing sequenced and acknowledged data packet and further being arranged to communicate said second tier data using a second protocol employing unacknowledged data packets.

7. An exercise system as claimed in claim 6 in which components of the first and second tier data are communicated using both protocols.

8. An exercise system as claimed in claim 6 or claim 7 in which the network is a local area network, a wide area network or the internet.

9. An exercise system as claimed in any one of claims 6 to 8 in which the first protocol is the transmission control protocol and the second protocol is a user datagram protocol.

10. An exercise system as claimed in any one of claims 6 to 9 further comprising a database (4) with which the central processor is ranged to communicate to store or retrieve information relating, for example, to users of said exercise devices, parameters relating to said exercise procedure and historal data relating to previous exercise procedures.

11. A method of controlling a plurality of mechanical exercise devices (1) to operate in a predetermined exercise procedure, the exercise devices each including sensors to provide signals representing selected performance parameters of the device and transducers responsive to control signals for setting selected operating parameters of the device, the exercise devices each having a local processor (2) connected to said exercise device to receive said sensor signals and to generate the sensor control signal, comprising the steps of
providing a means of communication between the local processor over a network with a remote central processor (3), whereby the central processor communicates with the plurality of local processors to coordinate the control of the plurality of exercise devices to operate in the exercise procedure,
characterised in that the local processors communicate with the remote central processor first tier data comprising at least data sent to the central processor identifying the local processor, data sent to the local processor defining the essential parameters of the said exercise procedure, data sent to the central processor defining final result parameters of the exercise procedure performed on the respective exercise devices, and data sent to the local processor defining final result parameters of the exercise procedure performed on the other exercise devices,
the local processor communicating with the remote central processor second tier data comprising at least data sent to the central processor during the course of the exercise procedure representing intermediate parameters of the exercise procedure performed on the respective exercise devices, and data sent to the local processor during the course of the exercise procedure representing said intermediate parameters of the exercise procedure performed on the other exercise devices,
wherein the local processor is arranged to communicate said first tier data using a first protocol employing sequenced and acknowledged data packets and is arranged to communicate said second tier data user using a second protocol employing unacknowledged data packets.

12. A method as claimed in claim 11 in which components of the first and second tier data are communicated using both protocols.

13. A method as claimed in claim 11 or claim 12 in which the network is a local area network, a wide area network or the internet.

14. A method as claimed in any one of claims 11 to 13 in which the first protocol is the transmission control protocol and the second protocol is the user datagram protocol.

15. A method as claimed in any one of claims 11 to 14 further comprising the step of providing communication between a database (4) and the central processor in which information may be stored or from which it may be retrieved relating, for example, to users of said exercise devices, parameters relating to said exercise procedure and historical data relating to previous exercise procedures.
